# EUROPEAN PATENT SPECIFICATION

(11) **EP 3 821 293 B1**
(45) Date of publication and mention of the grant of the patent: **07.02.2024**
(21) Application number: 19748476.9
(22) Date of filing: 10.07.2019
(51) Int. Cl.: G02C 7/04, G02C 7/10

(54) **LENS**
LINSE
LENTILLE

(30) Priority: 10.07.2018 GB 201811317
(43) Date of publication of application: 19.05.2021
(73) Proprietor: City, University of London, London EC1V 0HB (GB)
(72) Inventor: BARBUR, John, Northampton Square London EC1V 0HB (GB)
(74) Representative: Milhench, Mark Lorne
(86) International application number: PCT/EP2019/068556
(87) International publication number: WO 2020/011862

(56) References cited:
- WO-A1-2018/022735
- US-A- 5 774 202
- US-A1- 2010 103 371
- US-B1- 6 305 801
- US-B2- 7 106 509

## Description

This invention relates to lenses, particularly but not exclusively to contact lenses. Illustrative embodiments of the teachings of the invention relate to contact lenses that are operable to enhance a wearer's spatial vision. Other implementations of the teachings of the invention could provide an intra-ocular lens (iOL), and as such, references hereafter to "lens" should be construed accordingly.

US Patent No. 6305801 discloses a contact lens which has at least one optical filter region that can be surrounded by a clear optical region. The one or more filter regions have a diameter such that an increasing percentage of visible light is transmitted to the pupil of the eye as lighting conditions change from high-intensity lighting environments to low-intensity lighting environments. The cooperation between the changing pupil and lens provides an automatic adjustment of light intensity perceived by the wearer in response to the changing light conditions. Different filtering characteristics are provided in the filter regions to enhance perception of light corresponding to light reflected by an object or background scene used in outdoor sporting and recreational activities, while attenuating other reflected light.

US Patent No. 7106509 discloses that the selective filtering of light by polarization interference may be used to enhance vision and/or protect eyes from harmful light rays. For example, such filtering may be used in sunglasses, color corrective eyewear or protective eyewear. The selective filtering of incident light may provide any desired spectral transmission (including visible light and light not visible to the eye) and is performed by a pair of polarizing elements that sandwich a retarder stack. The filtering structure may be formed by multi-layer polarizing structures and may be formed by fabricating sheet laminates that are die cut to form inexpensive laminates. The laminates may be flat or curved in one (e.g., wrap-around) or more dimensions.

International PCT Patent Application No. WO2018/022735 teaches that enhanced visual acuity is provided by one or more exceptionally narrowband absorption dyes having sharp turn-on and cut-off slopes selected to spectrally sculpt light to correct most types of colorblindness, to exaggerate color contrast vision for everyone, and/or to markedly sharpen visual focus beyond normal vision. The dyes are incorporated, for example, in sunglasses, prescription and non-prescription eyewear, window thin films and artificial light sources such as incandescent, fluorescent, and LED lightbulbs to provide improved photopic color vision, improved mesopic dim light color vision, and also sharper scotopic night vision.

US patent No. 2010103371 discloses a contact lens with a central zone and one or more annular zones comprising bandpass filters to reduce chromatic blur.

In accordance with a presently preferred embodiment of the present invention, there is provided a lens as defined in Claim 1. Optional features of the lens defined in Claim 1 are set out in the dependent claims.

Arrangements embodying the teachings of the invention will now be described in detail with reference to the accompanying drawings, in which:
Fig. 1(a) is a schematic representation of light transmittance of a contact lens embodying the teachings of the invention;
Fig. 1(b) is a schematic representation of the lens depicted in Fig. 1(a) as it would appear when worn by a subject;
Fig. 2 is a schematic depiction (on the left of the figure) of four photoreceptors - namely L, M and S (cones) and R (rods), and (on the right of the figure) the spectral response of each photoreceptor to light;
Fig. 3 is a schematic representation of vision channels in human vision;
Fig. 4(a) is a schematic representation of the equivalent refractive error of the human eye as a function of the wavelength of light;
Fig. 4(b) is a chart that schematically depicts the spectral responsivity functions of the three classes of cone photoreceptor in the eye;
Fig. 5 is a chart that schematically depicts the Mean RMS aberrations for 26 subjects; and
Fig. 6 is a schematic illustration of illustrative centre and surround spectral transmittance functions for the centre and peripheral regions of the lens depicted schematically in Fig. 1.

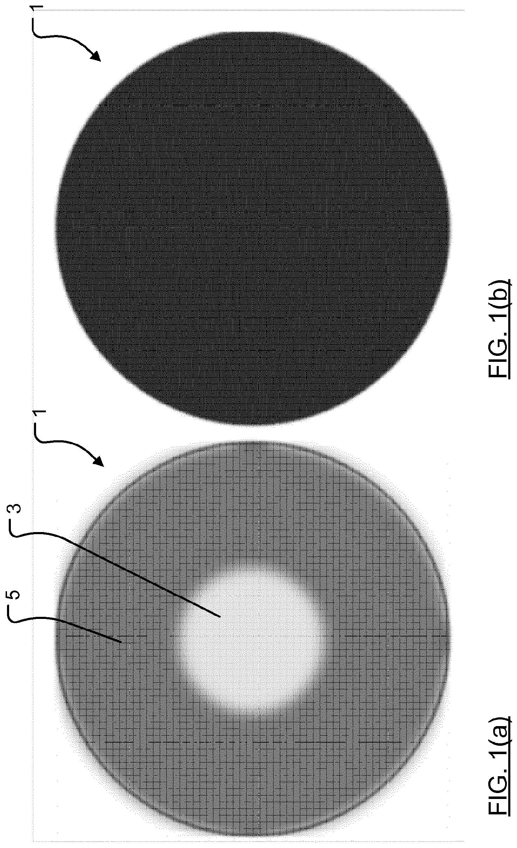

Arrangements embodying the teachings of the invention will now be described, by way of example, with specific reference to a contact lens 1 as depicted in Fig. 1.

It has transpired that significant improvement in vision (i.e., visual acuity, ability to see fine detail, depth of focus, etc.) can be achieved by selective *spectral* filtering of the light over the corneal surface before it enters the eye. This document describes a preferred implementation of the teachings of the invention and outlines the advantages of wearing a lens, for example a contact lens, that implements selective spectral filtering. Such a lens 1 is shown schematically in Fig. 1(a) and comprises a central region 3 which transmits yellow light and an annular outer region 5 that transmits blue light, or in other words a central region that is spectrally selective for light of wavelengths where (only) L and M cones have good spectral responsivity, and an outer region that is spectrally selective for light of wavelengths where (only) S cones have good spectral responsivity.

Advantages associated with embodiments of the present invention will now be justified using known facts about chromatic and monochromatic aberrations in the human eye, the contribution the signals generated in the four classes of photoreceptors in the retina make to our ability to see fine detail and faint objects, and the directional sensitivity of cones. The ability to see red/green (RG) and yellow/blue (YB) colour differences and mesopic/scotopic vision (i.e., ability to maintain useful vision at lower light levels) shall also be considered together with the decreased diffraction effects produced when using spectral absorption techniques to limit the effective pupil size aperture.

As described in detail below, the teachings of the present invention will greatly improve spatial vision without affecting significantly any other aspect of visual performance. In addition, the teachings of the invention provide an improvement in depth of field, particularly in the high mesopic range, with additional benefits to early presbyopes.

A presently preferred illustrative implementation of the teachings of the invention is shown schematically in Fig. 1a. The lens 1 is used to correct unwanted refractive errors and to block out UV light (as is done with conventional lenses). In addition, the lens 1 also filters out selectively short wavelength light over the centre disc 3 and middle and long wavelength light over its remaining outer regions 5. This means that the lens transmits only substantially 'yellow' light in the centre and substantially 'blue' light in the periphery. In spite of this spectrally selective light absorption, the pupil appears 'black' (to observers) as illustrated in Fig. 1b when the lens is worn by a subject. In a preferred implementation of the teachings of the invention, the centre disc 1 is in the region of 2 to 4 mm, preferably 2 to 3 mm and more preferably c. 3 mm in diameter; and the outer annulus 5 is in the region of 6 to 9 mm, more preferably c. 8 mm in diameter.

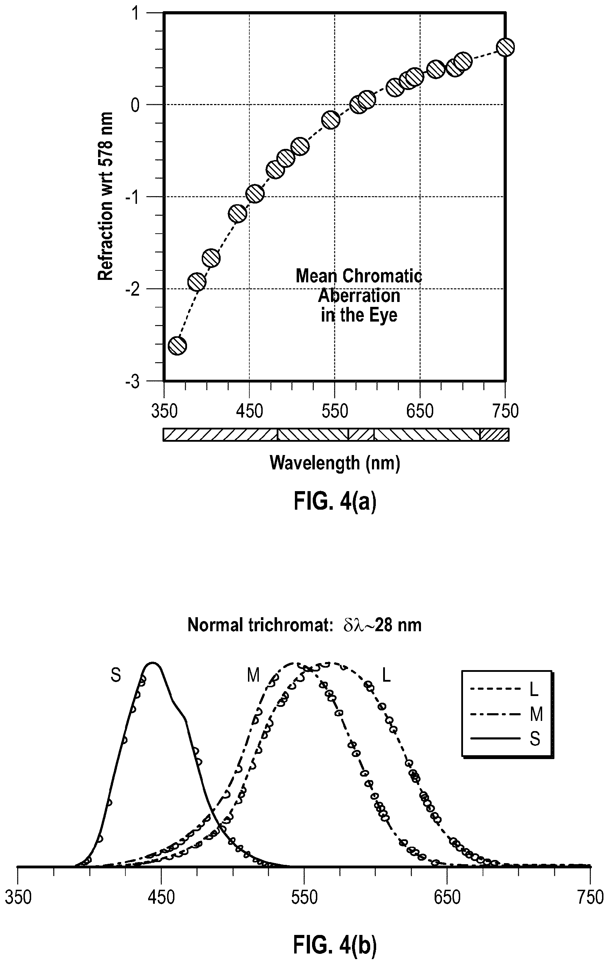

The great majority of normal eyes are almost 'diffraction' limited for pupil sizes below 2.5 ~ 3 mm. Higher order aberrations which limit the spatial resolution of the eye increase rapidly for pupil sizes larger than 3 mm and can be very large when the pupil size exceeds 6 mm - as shown schematically in Fig. 4. Both monochromatic and chromatic aberrations degrade retinal image quality and hence visual performance. One way of reducing the effect of higher order aberrations (particularly spherical aberration) on retinal image quality is to use only the central 2 to 3 mm of the pupil, but the use of small artificial pupils is impractical, although the proposed spectral absorption technique to reduce the "effective" pupil size also reduces the usual diffraction effects associated with small pupils. This diffraction reduction technique has other applications and is not limited solely to the present invention. The preferred embodiment depicted in Fig. 1a addresses these problems.

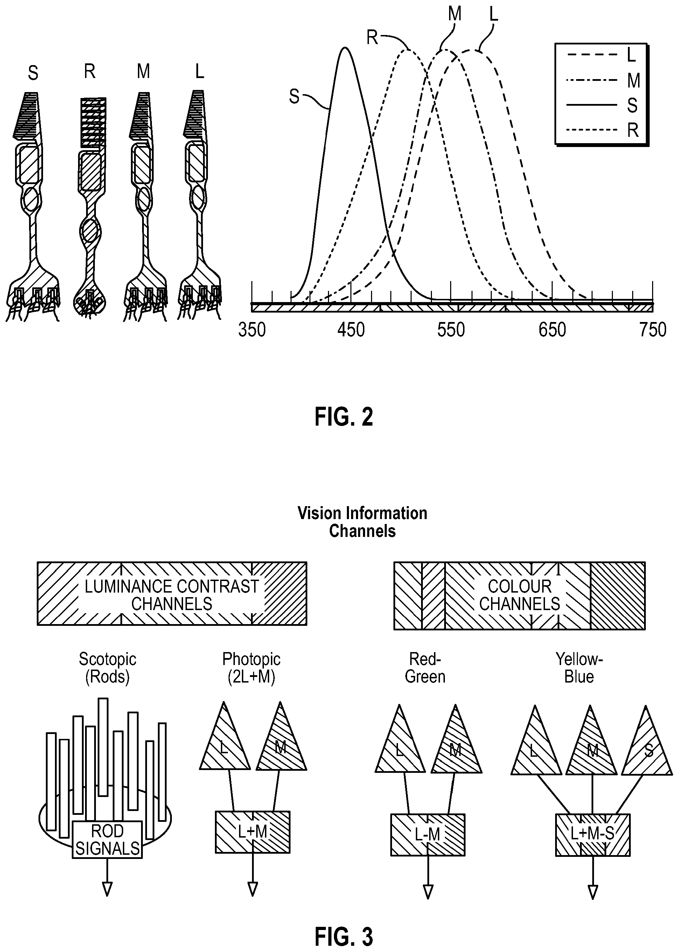

Referring now to Fig. 3, the luminance contrast channel mediates high resolution spatial vision and relies mostly on long and middle wavelength light (i.e., 'red' and 'green'). Rod mediated vision and yellow / blue (YB) colour vision make use of short wavelength ('blue') light, but these channels have poor spatial resolution and do not therefore require sharp retinal images. In embodiment incorporating the teachings of the invention, these channels will receive the 'blue' light they need only from the outer regions of the pupil. Chromatic aberration in the eye can be very large and when the long wavelength image is in optimum focus on the retina, the short wavelength image is subjected to large refractive error. The latter is less important for either YB or rod mediated vision, but is important for acuity and detection of fine detail in low contrast. The preferred embodiment of lens disclosed herein causes a significant reduction in both monochromatic and chromatic aberrations in the eye, thus yielding improved spatial vision, without affecting significantly either YB colour vision or rod-mediated vision. The use of a 'spectrally tuned' small pupil has other advantages in terms of reducing, and in some instances eliminating, the effects of diffraction for small pupils and at the same time by increasing the depth of field. The latter may be of significant advantage in early presbyopes.

The human eye tends to exhibit large amounts of chromatic aberration as the wavelength changes from blue light (just above 400 nm) to red light (below 780nm). In terms of refractive error this is estimated to exceed 2.5 dioptres. We are somewhat protected against the full chromatic aberration in the eye simply because our high acuity, spatial vision relies only on L and M cone signals.

Referring now to Fig. 2, the four photoreceptors shown on the left correspond to L, M and S (cones) and R (rods). Each photoreceptor contains a photosensitive pigment which responds best to light of different wavelengths as shown on the right of the figure. The wavelengths of peak spectral responsivity are S (442), R (505), M (542) and L (570) nm. The photopic luminance channel sums up signals from L and M cones (Fig. 2) and mediates high resolution, achromatic vision and enables us to see fine spatial details in low contrast. Chromatic aberration, largely within the lens of the eye causes large refractive errors (Fig. 4a). Although the peak spectral responsivities of L and M cones are separated only by ~ 28 nm (which corresponds to less than 0.3D of chromatic aberration), the M-cones continue to respond well down to 450nm and L-cones extend to 660 nm. The corresponding chromatic aberration between these extremes can be as large as ~ 1.3D.

The peak spectral responsivities of L and M cones are separated only by ~ 28 nm (which corresponds to less than 0.3D of chromatic aberration). This value is only fractionally larger than the random fluctuations that drive the accommodation mechanism and can easily be tolerated by the eye.

M-cones do however continue to respond well down to 450nm and L-cones extend to 660 nm. The corresponding chromatic aberration between these extreme wavelengths can be as large as ~ 1.3D and this larger value can have a detrimental effect on the luminance contrast channel and hence our ability to see fine detail in 'white' light (Fig. 4).

The lens of the preferred embodiment reduces the damaging effects of chromatic aberration on retinal image quality in the luminance contrast channel by reducing the amount of aberrated, short wavelength light that would normally be absorbed by M cones. Light from the sharply defined image formed by 'yellow' light that passes through the centre of the pupil is likely to be very effective for L and M cones because of very small Stiles Crawford effect (i.e., maximum directional sensitivity of cones when small pupils are involved) and the reduced chromatic and monochromatic aberrations associated with this small, central region of the pupil.

Fig. 3 is a schematic representation of vision channels in human vision. The 'scotopic' luminance channel relies on rod signals and has very poor spatial resolution. The 'photopic' luminance channel relies on the sum of L and M cone signals and has high spatial resolution and responds best to middle and long wavelength light. The YB colour channel relies on S-cone signals which have very low spatial resolution and respond best to short wavelength light. The RG colour channel relies only on signals from L and M cones.

There are four spectrally tuned photosensitive pigments in cone and rod photoreceptors in the retina. Each photoreceptor contains a photosensitive pigment which responds best to light of different wavelengths as shown in Fig. 2.

The wavelengths of peak spectral responsivity are S (442), R (505), M (542) and L (570) nm. The photopic luminance channel sums up signals from L and M cones (Fig. 2) and mediates high resolution, achromatic vision and enables us to see fine spatial details in low contrast.

Rod photoreceptors tend to have high sensitivity to light, but very poor spatial resolution and low spatial contrast sensitivity. They form the 'scotopic' luminance channel. Rod vision relies mostly on short-wavelength (blue) light and is tolerant of poor retinal image quality. The lens of the preferred embodiment improves the output of the luminance contrast channel by providing improved image quality in 'yellow' light without affecting significantly either YB or rod mediated vision.

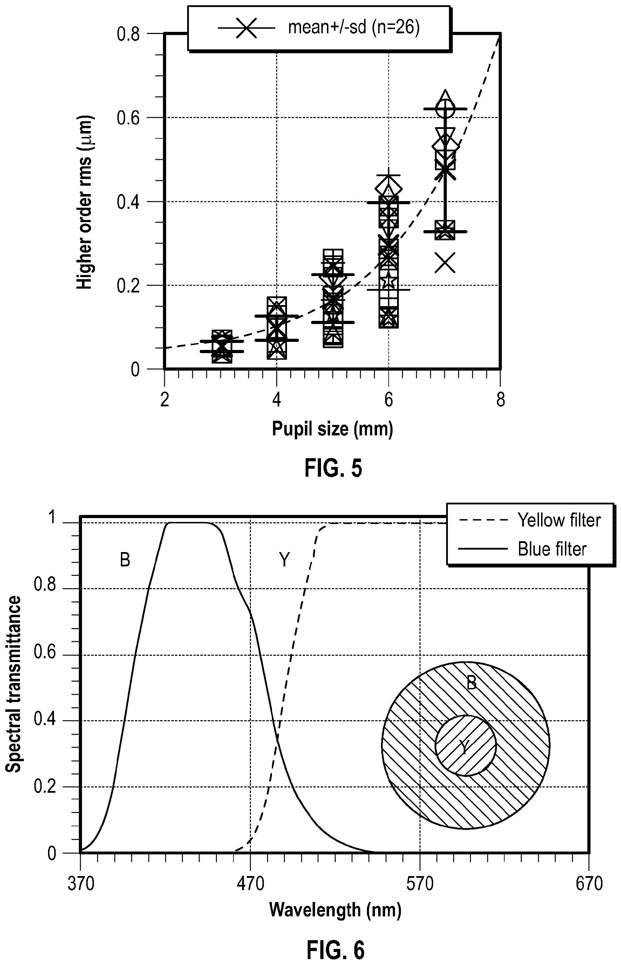

In the human eye, rays derived from the outer regions of the pupil (after passing through the cornea and the lens) tend to be more aberrated than the paraxial rays that pass through the centre of the pupil. Spherical aberration (SA) affects the quality of the image formed on the retina, both centrally as well as in the more distant regions away from the point of regard (the latter corresponds to the region that yields the highest spatial vision, the foveola). Wavefront spherical aberration increases with the forth power of ray height and can have devastating effects on image quality for very large pupils in eyes with large amounts of SA. When only cone vision is involved the directional sensitivity of cones offer some protection since cones are less sensitive to rays that enter the eye through the more peripheral regions of the pupil, but this protection is not sufficient in eyes with large pupils and hence SA. Coma is the next important aberration which increases with the third power of ray height. A measure of the expected effect of aberrations in the eye as a function of the size of the pupil is the RMS wavefront aberration. This parameter has been measured in a number of subjects in many studies as a function of pupil size and the results are similar to those shown in Fig. 5.

The important conclusions to draw from these results is that many eyes are almost 'diffraction' limited for pupil sizes ~ 2.5 to 3 mm and that the higher order aberrations increase rapidly afterwards with increasing pupil size. One way of reducing the effect of higher order aberrations on retinal image quality is to use only the central 2 to 3 mm of the pupil, although the use of small artificial pupils causes an increase in diffraction, which in turn can reduce image quality.

Referring to Fig. 6, there is depicted an illustration of centre and surround spectral transmittance functions for the centre region 3 (yellow) and for the peripheral region 5 (blue) of the lens 1 disclosed herein for enhancing spatial vision. An embodiment may incorporate a UV blocking filter (as shown). The cut off regions for transmission of 'blue' and 'green' light and the size of the centre (yellow) region are shown schematically and may vary. The preferred implementation may be particularly suitable for use whilst mountaineering or skiing.

The presently preferred implementation of the teachings of the invention reduces increased diffraction through selective absorption of unwanted photons before these pass through the lens. The preferred implementation also reduces monochromatic and chromatic ocular aberrations to negligible levels and increases the depth of field. The preferred implementation may turn out to be particularly useful in early presbyopia by reducing the effect of residual defocus. Colour vision is unlikely to be affected significantly since the 'white balance' is maintained by allowing short wavelength light to pass through the outer regions of the lens. Typical functions of an illustrative implementation are illustrated schematically in Fig. 6.

An additional advantage of the preferred embodiment is the increased safety that is achieved by decreasing the amount of short wavelength light that reaches the retina. It has been claimed that long term use of 'blue' filtering IOLs protects the retina against blue light damage. Reducing much of the short wavelength light may however alter YB chromatic sensitivity, affect normal pupil responses to light as well as circadian rhythms which rely mostly on short wavelength light. The preferred embodiment offers an excellent compromise by limiting the amount of short wavelength light reaching the retina without affecting significantly either colour vision or circadian rhythms.

In summary, embodiments described herein provide reduced diffraction (as compared with the levels of diffraction that would occur with a small pupil) due to photons being absorbed instead of scattered. In addition, the relatively small size of the first zone reduces monochromatic aberrations as one would expect for a small pupil. In addition, chromatic aberration is reduced because red and green light (passed by the first zone) are narrowly spaced in the electromagnetic spectrum. Lastly, the so-called 'pinhole' effect associated with the small first zone improves image quality and increases depth of field. One direct application of this last effect is that persons with presbyopia are likely to see better at shorter distances than they would without lenses. It is also anticipated that a lens embodying the teachings of the invention will be of benefit to patients with keratoconus, who exhibit much greater spherical aberration with increasing pupil size.

The aforementioned benefits accrue without significantly affecting yellow/blue vision, without significantly affecting red/green vision, without appreciably affecting circadian rhythm (and potentially rod / melanopsin related control of pupil size), and with only a small reduction in retinal illuminance.

It will be appreciated that whilst various aspects and embodiments of the present invention have heretofore been described, the scope of the present invention is not limited to the particular arrangements set out herein and instead extends to encompass all arrangements, and modifications and alterations thereto, which fall within the scope of the appended claims.

## Claims

1. A lens (1) for enhancing spatial vision in humans, said lens (1) comprising a contact lens or an intraocular lens and including a first approximately central disc-shaped region (3) that is spectrally selective for light of a first band of wavelengths where L and M cones of a human eye have good spectral responsivity, and a second approximately annular region (5) extending outwardly from said first region that is spectrally selective for light of a second band of wavelengths where S cones of a human eye have good spectral responsivity.

2. A lens according to Claim 1, wherein the first and second bands at least partly overlap.

3. A lens according to Claim 1, wherein the first and second bands are discrete from one another.

4. A lens according to any preceding claim, wherein the lens (1) is configured so that the first (3) and second (5) regions pass only the first and second wavelength bands, respectively.

5. A lens according to any preceding claim, wherein the first region (3) is configured to filter out shorter wavelengths of incident light, and the second region (5) is configured to filter out longer wavelengths of incident light.

6. A lens according to Claim 5, wherein the first region (3) is configured to pass light of wavelengths above about 490 nm.

7. A lens according to Claim 6, wherein the first region (3) has a transmittance increasing from about 0.5 at 490 nm to approaching 1 at higher wavelengths.

8. A lens according to any preceding claim, wherein the second region (5) is configured to pass light having a wavelength between about 370 to 480 nm.

9. A lens according to Claim 8, wherein the second region (5) has a transmittance increasing from about 0.5 at 400 nm to approaching 1 and reducing again to about 0.5 at roughly 480nm.

10. A lens according to any preceding claim, wherein the second region (5) has a transmittance of approximately zero below 370 nm and thus filters out incident ultraviolet light.

11. A lens according to any preceding claim, wherein the first region (3) is roughly 2 - 3.5 mm in diameter, and the second region (5) extends from the first region towards a peripheral edge of the lens (1).

12. A lens according to Claim 11, wherein the first region (3) is approximately 3 mm in diameter.

13. A lens according to Claim 11, wherein the second region (5) has a diameter of roughly 8 - 10 mm.

14. A lens according to Claim 13, wherein the second region (5) has a diameter of approximately 8 mm.

## Patentansprüche

1. Linse (1) zur Verbesserung des räumlichen Sehens bei Menschen, wobei die Linse (1) eine Kontaktlinse oder eine Intraokularlinse umfasst und einen ersten, etwa zentralen scheibenförmigen Bereich (3), der spektral selektiv für Licht eines ersten Wellenlängenbandes ist wobei die L- und M-Zapfen eines menschlichen Auges eine gute spektrale Empfindlichkeit aufweisen, und einen zweiten etwa ringförmigen Bereich (5) enthält, der sich vom ersten Bereich nach außen erstreckt und spektral selektiv für Licht eines zweiten Wellenlängenbandes ist, bei dem die S-Zapfen eines menschlichen Auges eine gute spektrale Empfindlichkeit aufweisen.

2. Linse nach Anspruch 1, wobei sich das erste und das zweite Band zumindest teilweise überlappen.

3. Linse nach Anspruch 1, wobei das erste und das zweite Band voneinander separat sind.

4. Linse nach einem der vorhergehenden Ansprüche, wobei die Linse (1) so konfiguriert ist, dass der erste (3) und der zweite (5) Bereich nur das erste bzw. zweite Wellenlängenband durchlassen.

5. Linse nach einem der vorhergehenden Ansprüche, wobei der erste Bereich (3) so konfiguriert ist, dass er kürzere Wellenlängen von einfallendem Licht herausfiltert, und der zweite Bereich (5) so konfiguriert ist, dass er längere Wellenlängen von einfallendem Licht herausfiltert.

6. Linse nach Anspruch 5, wobei der erste Bereich (3) so konfiguriert ist, dass er Licht mit Wellenlängen über etwa 490 nm durchlässt.

7. Linse nach Anspruch 6, wobei der erste Bereich (3) einen Transmissionsgrad aufweist, der von etwa 0,5 bei 490 nm auf nahezu 1 bei höheren Wellenlängen ansteigt.

8. Linse nach einem der vorhergehenden Ansprüche, wobei der zweite Bereich (5) so konfiguriert ist, dass er Licht mit einer Wellenlänge zwischen etwa 370 und 480 nm durchlässt.

9. Linse nach Anspruch 8, wobei der zweite Bereich (5) einen Transmissionsgrad aufweist, der von etwa 0,5 bei 400 nm auf nahezu 1 ansteigt und wieder auf etwa 0,5 bei annähernd 480 nm abnimmt.

10. Linse nach einem der vorhergehenden Ansprüche, wobei der zweite Bereich (5) einen Transmissionsgrad von etwa Null unter 370 nm aufweist und somit einfallendes ultraviolettes Licht herausfiltert.

11. Linse nach einem der vorhergehenden Ansprüche, wobei der erste Bereich (3) einen Durchmesser von annähernd 2 bis 3,5 mm hat und sich der zweite Bereich (5) vom ersten Bereich in Richtung einer Umfangskante der Linse (1) erstreckt.

12. Linse nach Anspruch 11, wobei der erste Bereich (3) einen Durchmesser von etwa 3 mm hat.

13. Linse nach Anspruch 11, wobei der zweite Bereich (5) einen Durchmesser von annähernd 8 bis 10 mm aufweist.

14. Linse nach Anspruch 13, wobei der zweite Bereich (5) einen Durchmesser von etwa 8 mm aufweist.

## Revendications

1. Lentille (1) destinée à améliorer la vision spatiale chez les humains, ladite lentille (1) comprenant une lentille de contact ou une lentille intraoculaire et comprenant une première région approximativement centrale en forme de disque (3) qui est spectralement sélective pour la lumière d'une première bande de longueurs d'onde où les cônes L et M d'un oeil humain comportent une bonne réactivité spectrale, et une seconde région approximativement annulaire (5) s'étendant vers l'extérieur de ladite première région qui est spectralement sélective pour la lumière d'une seconde bande de longueurs d'onde où les cônes S d'un oeil humain comportent une bonne réactivité spectrale.

2. Lentille selon la revendication 1, dans laquelle les première et seconde bandes se chevauchent au moins partiellement.

3. Lentille selon la revendication 1, dans laquelle les première et seconde bandes sont distinctes l'une de l'autre.

4. Lentille selon l'une quelconque des revendications précédentes, dans laquelle la lentille (1) est configurée de telle sorte que les première (3) et seconde (5) régions laissent passer uniquement les première et seconde bandes de longueurs d'onde, respectivement.

5. Lentille selon l'une quelconque des revendications précédentes, dans laquelle la première région (3) est configurée pour filtrer les longueurs d'onde plus courtes de lumière incidente, et la seconde région (5) est configurée pour filtrer les longueurs d'onde plus longues de lumière incidente.

6. Lentille selon la revendication 5, dans laquelle la première région (3) est configurée pour laisser passer la lumière de longueurs d'onde supérieures à environ 490 nm.

7. Lentille selon la revendication 6, dans laquelle la première région (3) comporte une transmission qui augmente d'environ 0,5 à 490 nm jusqu'à s'approcher de 1 à des longueurs d'onde plus élevées.

8. Lentille selon l'une quelconque des revendications précédentes, dans laquelle la seconde région (5) est configurée pour laisser passer la lumière comportant une longueur d'onde comprise entre environ 370 et 480 nm.

9. Lentille selon la revendication 8, dans laquelle la seconde région (5) comporte une transmission qui augmente d'environ 0,5 à 400 nm jusqu'à s'approcher de 1 et qui diminue à nouveau jusqu'à environ 0,5 à environ 480 nm.

10. Lentille selon l'une quelconque des revendications précédentes, dans laquelle la seconde région (5) comporte une transmission approximativement nulle en dessous de 370 nm et filtre ainsi la lumière ultraviolette incidente.

11. Lentille selon l'une quelconque des revendications précédentes, dans laquelle la première région (3) a un diamètre d'environ 2 à 3,5 mm, et la seconde région (5) s'étend depuis la première région vers un bord périphérique de la lentille (1).

12. Lentille selon la revendication 11, dans laquelle la première région (3) a un diamètre d'environ 3 mm.

13. Lentille selon la revendication 11, dans laquelle la seconde région (5) comporte un diamètre d'environ 8 à 10 mm.

14. Lentille selon la revendication 13, dans laquelle la seconde région (5) comporte un diamètre d'environ 8 mm.
